# EUROPEAN PATENT APPLICATION

(11) **EP 0 963 989 A1**
(43) Date of publication of application: **15.12.1999**
(21) Application number: 98938886.3
(22) Date of filing: 19.08.1998
(51) Int. Cl.: C07D 501/12, C07D 501/24

(54) **CEPHALOSPORIN CRYSTALS AND PROCESS FOR PRODUCING THE SAME**

(30) Priority: 25.08.1997 JP 22814797
(71) Applicant: OTSUKA CHEMICAL COMPANY, LIMITED, Osaka-shi, Osaka 540-0021 (JP)
(72) Inventor: KAMEYAMA, Yutaka, Tokushima-shi Tokushima 771-0134 (JP); SHIROI, Takashi, Itano-gun Tokushima 771-0204 (JP)
(74) Representative: Barz, Peter
(86) International application number: JP9803664
(87) International publication number: WO9910352

(57) **Abstract**

The object of the invention is to provide cephalosporin crystals which are highly stable for a long period under economical storage conditions. The cephalosporin crystals of the invention are obtainable by crystallizing an oily cephalosporin in a solvent containing an alcohol.

## Description

### TECHNICAL FIELD

The present invention relates to cephalosporin crystals and processes for their preparation.

### BACKGROUND ART

Cephalosporins, which are represented by the formula: 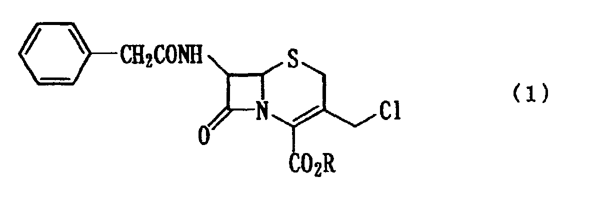 wherein R represents p-methoxybenzyl or diphenylmethyl, are compounds known as important intermediates for synthesis of various antibiotics such as cefazolin.

Cefazolin is an antibiotic widely used today and represented by the formula: 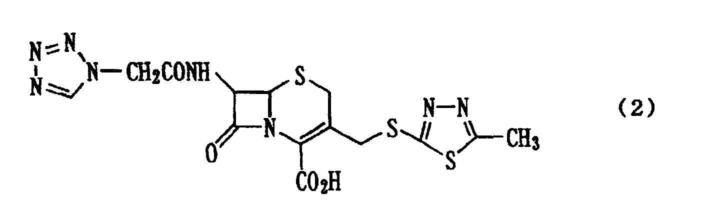 (see "Saishin Koseibusshitsu Yoran" by Katsuharu Sakai, p. 59).

Cephalosporins represented by the formula (1) are conventionally produced by, for example, the method disclosed in Japanese Examined Patent Publication No. 41153/1989. In the method, the desired cephalosporin compound is obtained only in the form of oils, although purified through a silica gel column.

Since the cephalosporin of the formula (1) has a reactive chlorine atom in the molecule, it is unstable in an oil form. For example, when stored at room temperature, it releases hydrochloric acid which accelerates self-decomposition, resulting in deteriorated quality. Thus, it has been desired to provide cephalosporins in a form stable to handling for a relatively long period under moderate conditions.

### DISCLOSURE OF THE INVENTION

An object of the present invention is to provide a cephalosporin which is highly stable for a long period under economical storage conditions.

Another object of the invention is to provide a cephalosporin in the form of crystals which are stable to handling for a long period under moderate conditions.

A further object of the invention is to provide a process for preparing the cephalosporin crystals.

Other features of the invention will be apparent from the following description.

The present inventors did extensive research to accomplish the above objects, and found that a cephalosporin in the form of stable crystals can be obtained by crystallizing an oily cephalosporin in a solvent containing an alcohol. The present invention has been accomplished based on this novel finding.

The present invention provides cephalosporin crystals represented by the formula (1).

The present invention further provides a process for preparing cephalosporin crystals comprising the step of crystallizing, in a solvent containing an alcohol, an oily cephalosporin represented by the formula: 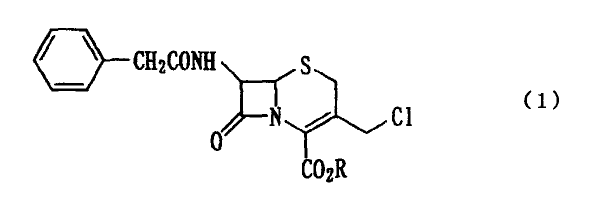 wherein R is as defined above.

The cephalosporin crystals of the present invention are not liable to deteriorate in quality, since they do not release hydrochloric acid which accelerates self-decomposition, even if stored at room temperature for a long period. Therefore, the cephalosporine crystals of the invention are highly stable for a long period under economical storage conditions, and can be handled under moderate conditions.

Specifically stated, the present invention provides cephalosporin crystals represented by the formula (1a): 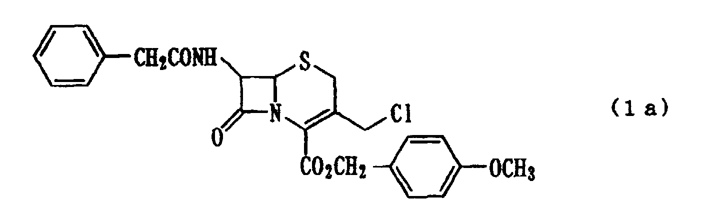 and cephalosporin crystals represented by the formula (1b):

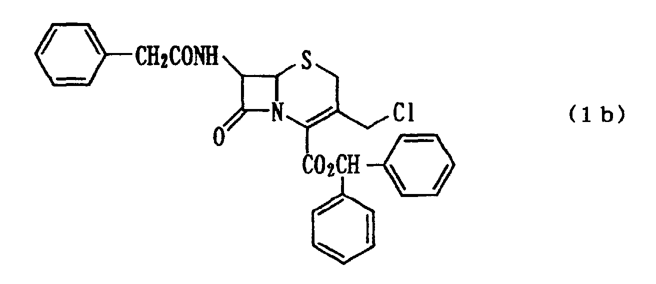

The cephalosporin crystals of the formula (1a) are white crystals characterized by the following X-ray powder diffraction pattern obtained by copper radiation of λ=1.5418 angstroms through a monochromator silk filter.

| d | I/Io |
|---|---|
| 12.94 - 12.96 | 0.55 - 0.67 |
| 11.67 - 11.69 | 0.46 - 0.56 |
| 9.90 - 9.92 | 0.09 - 0.11 |
| 6.46 - 6.48 | 0.57 - 0.69 |
| 6.32 - 6.34 | 0.22 - 0.26 |
| 5.80 - 5.82 | 0.56 - 0.68 |
| 4.95 - 4.97 | 0.42 - 0.52 |
| 4.72 - 4.74 | 0.63 - 0.77 |
| 4.65 - 4.67 | 0.65 - 0.79 |
| 4.50 - 4.52 | 0.60 - 0.74 |
| 4.43 - 4.45 | 0.28 - 0.34 |
| 4.28 - 4.30 | 0.50 - 0.62 |
| 4.16 - 4.18 | 1.00 |
| 4.04 - 4.06 | 0.23 - 0.28 |
| 3.97 - 3.99 | 0.15 - 0.19 |
| 3.85 - 3.87 | 0.67 - 0.81 |
| 3.77 - 3.79 | 0.42 - 0.52 |
| 3.69 - 3.71 | 0.13 - 0.15 |
| 3.55 - 3.57 | 0.06 - 0.08 |
| 3.53 - 3.54 | 0.05 - 0.07 |
| 3.44 - 3.46 | 0.49 - 0.59 |
| 3.36 - 3.38 | 0.18 - 0.22 |
| 3.28 - 3.30 | 0.13 - 0.15 |
| 3.21 - 3.23 | 0.18 - 0.22 |
| 3.19 - 3.21 | 0 14 - 0.17 |
| 3.15 - 3.17 | 0.18 - 0.22 |
| 3.08 - 3.10 | 0.16 - 0.20 |
| 2.89 - 2.91 | 0.20 - 0.24 |
| 2.84 - 2.86 | 0.09 - 0.11 |
| 2.67 - 2.69 | 0.13 - 0.15 |
| 2.59 - 2.61 | 0.14 - 0.18 |
| 2.57 - 2.59 | 0.10 - 0.12 |
| 2.49 - 2.51 | 0.09 - 0.11 |
| 2.48 - 2.50 | 0.12 - 0.14 |

wherein d is the interplanar spacing, and I/Io is the relative intensity.

The cephalosporin crystals of the formula (1b) are white crystals characterized by the following X-ray powder diffraction pattern obtained by copper radiation of λ=1.5418 angstroms through a monochromator silk filter.

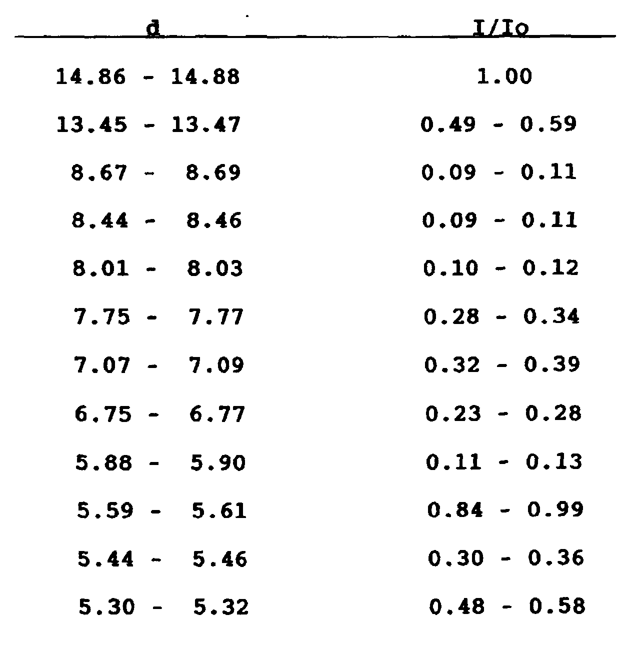

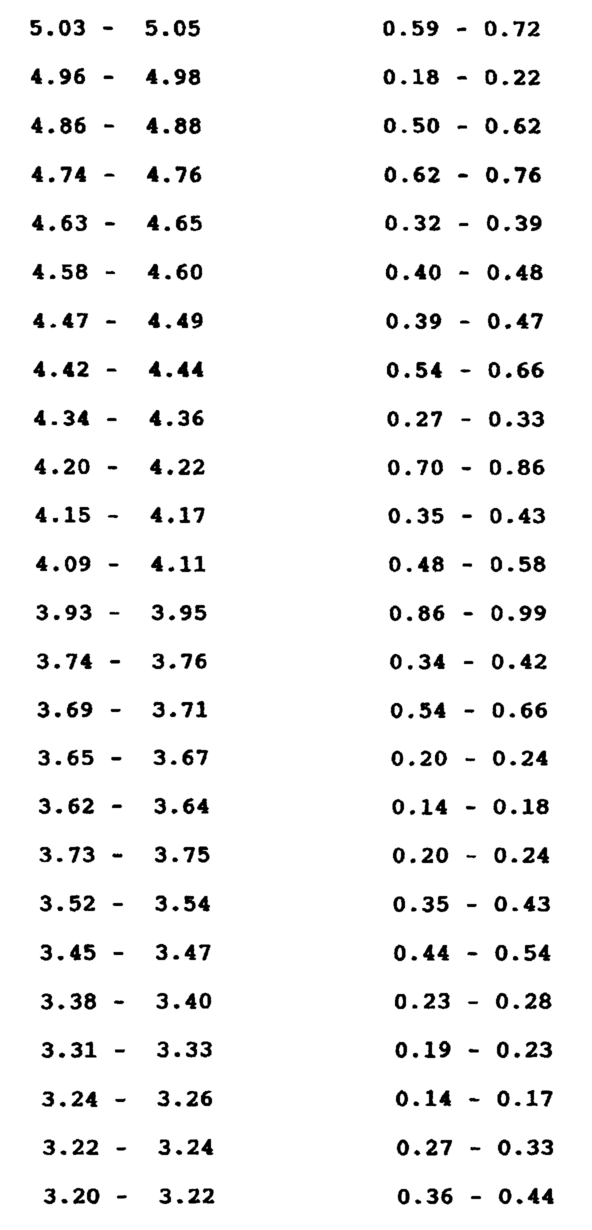

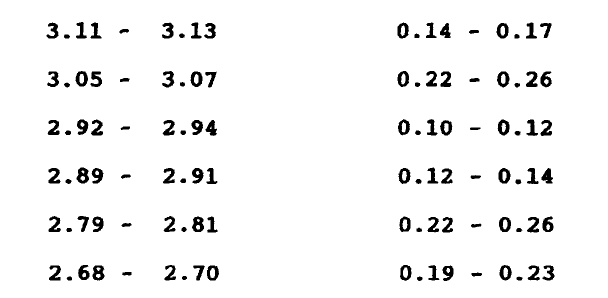

wherein d is the interplanar spacing, and I/Io is the relative intensity.

The cephalosporin crystals of the invention can be obtained by crystallizing an oily cephalosporin represented by the formula (1) in a solvent containing an alcohol. Specifically stated, the cephalosporin crystals can be prepared by dissolving an oily cephalosporin in a solvent, and adding the solution in an alcohol to precipitate crystals.

The solvent for dissolving the oily cephalosporin of the formula (1) may be any conventional solvent which is capable of dissolving the oily cephalosporin and which is compatible with alcohols. Examples of such solvents include amide solvents such as dimethylformamide and N-methylpyrrolidone, and ether solvents such as dioxane and tetrahydrofuran. The amount of the oily cephalosporin to be dissolved in the solvent is not limited.

Various alcohols are usable, among which aliphatic saturated lover alcohols are preferable, and methanol, ethanol, isopropanol and the like are particularly preferable.

The alcohol for use in the invention may contain water. The water content is usually 50% or less. preferably 30% or less, based on the amount of the alcohol and water combined.

When the solution of oily cephalosporin in solvent is added to the alcohol, the amount of the alcohol is not limited as long as cephalosporin crystals can be precipitated. Usually, however, the alcohol is used in an amount of 100 to 10000 wt. parts, preferably 200 to 3000 wt. parts, more preferably 400 to 1000 wt. parts, per 100 wt. parts of the solvent.

It is desirable that the alcohol be cooled before adding the solution to the alcohol. The temperature of the cooled alcohol is usually about -20 to about 15°C, preferably about 0 to about 5°C. Addition of the solution to an uncooled alcohol produces a highly polar component wherein the chlorine atom at the 3'-position of the cephalosporin of the formula (1) has been replaced with an alkozy group by reaction with the alcohol, or various decomposition products wherein the lactam ring of the cephalosporin of the formula (1) has opened. As the result, it becomes difficult to obtain the cephalosporin crystals of the formula (1) in a high yield. In contrast, use of a cooled alcohol inhibits production of the highly polar component wherein the chlorine atom at the 3'-position of the cephalosporin of the formula (1) has been replaced with an alkoxy group by reaction with the alcohol, and the various decomposition products wherein the lactam ring of the cephalosporin of the formula (1) has opened. As the result, remarkable effect, i.e., a high yield of the crystals of the cephalosporin of the formula (1), can be achieved. This matter is apparent from Comparative Test Examples 1 and 2 shown hereinafter.

The cephalosporin crystals of the invention thus obtained can be isolated and purified by conventional methods. For example, the cephalosporin crystals may be isolated by filtration and dried. Filtration and drying can be carried out by any conventional methods without limitations. Examples of filtration methods include filtration under atmospheric pressure, filtration under pressure, and centrifugation. Examples of drying methods include forced-air drying, tray drying and drying under reduced pressure. The drying temperature is usually 15 to 60°C. It is particularly preferable that the drying be carried out at about 25 to about 45°C under reduced pressure.

The cephalosporin crystals of the invention can be synthesized into cefazolin useful as an antibiotic, by, for example, the method illustrated by the following Reaction Scheme-1.

### Reaction Scheme-1:

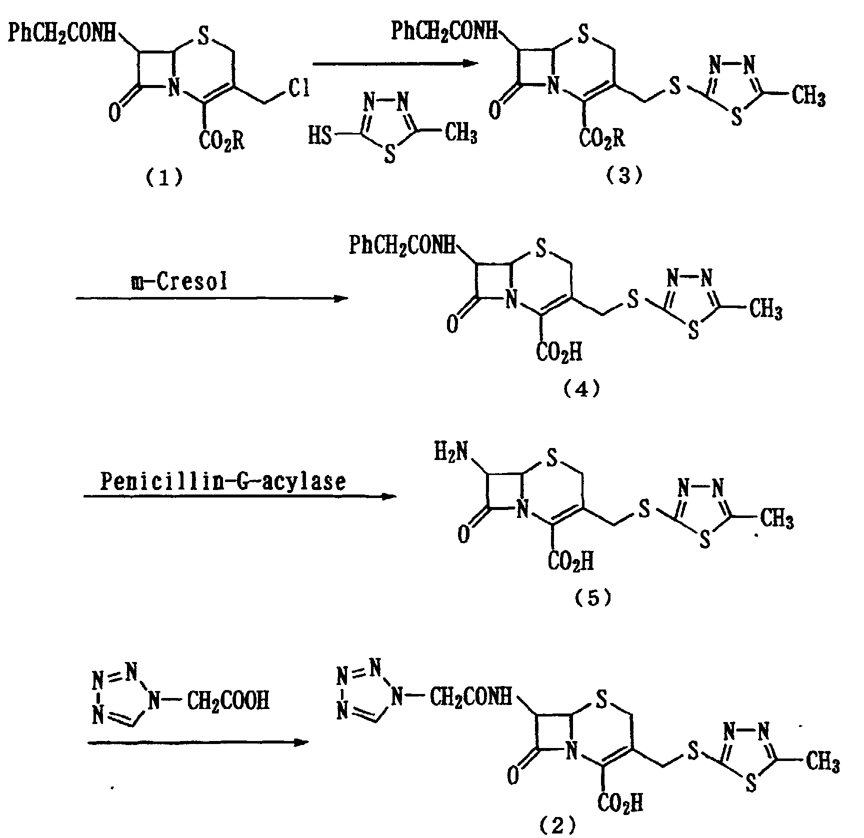

### BEST MODE FOR CARRYING OUT THE INVENTION

The following Examples, Comparative Test Examples, and Reference Examples illustrate the present invention in further detail.

### Example 1

600 ml of methanol was placed in a 1-liter, egg plant-type flask and cooled to 3°C. Separately, a solution was prepared by dissolving 120 g of oily 7-phenylacetamido-3-chloromethyl-cephem-4-carboxylic acid p-methoxybenzyl ester in 120 ml of dimethylformamide. The dimethylformamide solution was added dropwise to the methanol fully cooled. The mixture was stirred for 30 minutes to precipitate crystals of 7-phenylacetamido-3-chloromethyl-cephem-4-carboxylic acid p-methoxybenzyl ester. The precipitate was collected by filtration under reduced pressure, washed with methanol and dried under reduced pressure, giving 102 g of crystals of 7-phenylacetamido-3-chloromethyl-cephem-4-carboxylic acid p-methoxybenzyl ester (compound (1a)).

Shown below are the NMR spectrum and X-ray powder diffraction pattern of the obtained crystals. The X-ray powder diffraction pattern was obtained by copper radiation of λ=1.5418 angstroms through a monochromator silk filter.
¹H-NMR (CDCl₃) δ ppm;
3.41 (d, J=18Hz, 1H),
3.60 (d, J=18Hz, 1H),
3.60 (d, J=16Hz, 1H),
3.67 (d, J=16Hz, 1H),
3.80 (s, 3H),
4.39 (d, J=12Hz, 1H),
4.50 (d, J=12Hz, 1H),
4.92 (d, J=5Hz, 1H),
5.20 (d, 2H),
5.82 (dd, J=5Hz, J=9Hz, 1H),
6.08 (d, J=9Hz, 1H),
6.86-7.40 (m, 9H)

X-ray powder diffraction pattern;

| d | I/Io |
|---|---|
| 12.95 | 0.61 |
| 11.68 | 0.51 |
| 9.91 | 0.10 |
| 6.47 | 0.63 |
| 6.33 | 0.24 |
| 5.81 | 0.62 |
| 4.96 | 0.47 |
| 4.73 | 0.70 |
| 4.66 | 0.72 |
| 4.51 | 0.67 |
| 4.44 | 0.31 |
| 4.29 | 0.56 |
| 4.17 | 1.00 |
| 4.05 | 0.25 |
| 3.98 | 0.17 |
| 3.86 | 0.74 |
| 3.78 | 0.47 |
| 3.70 | 0.14 |
| 3.56 | 0.07 |
| 3.54 | 0.06 |
| 3.45 | 0.54 |
| 3.37 | 0.20 |
| 3.29 | 0.14 |
| 3.22 | 0.20 |
| 3.20 | 0.15 |
| 3.16 | 0.20 |
| 3.09 | 0.18 |
| 2.90 | 0.22 |
| 2.85 | 0.10 |
| 2.68 | 0.14 |
| 2.60 | 0.16 |
| 2.58 | 0.11 |
| 2.50 | 0.10 |
| 2.49 | 0.13 |

wherein d is the interplanar spacing, and I/Io is the relative intensity.

### Example 2

600 ml of methanol and 90 ml of water were placed in a 1-liter, egg plant-type flask and cooled to 3°C. Separately, a solution was prepared by dissolving 120 g of oily 7-phenylacetamido-3-chloromethyl-cephem-4-carboxylic acid diphenylmethyl ester in 120 ml of dimethylformamide. The dimethylformamide solution was added dropwise to the methanol/water mixture fully cooled. The resulting mixture was stirred for 30 minutes to precipitate crystals of 7-phenylacetamido-3-chloromethyl-cephem-4-carboxylic acid diphenylmethyl ester. The precipitate was collected by filtration under reduced pressure, washed with methanol and dried under reduced pressure, giving 96 g of crystals of 7-phenylacetamido-3-chloromethyl-cephem-4-carboxylic acid diphenylmethyl ester.

Shown below are the NMR spectrum and X-ray powder diffraction pattern of the obtained crystals. The X-ray powder diffraction pattern was obtained by copper radiation of λ=1.5418 angstrom through a monochrometer silk filter.
¹H-NMR (CDCl₃) δ ppm;
   3.40 (d, J=18Hz, 1H),
   3.55 (d, J=18Hz, 1H),
   3.59 (d, J=16Hz, 1H),
   3.66 (d, J=16Hz, 1H),
   4.36 (s, 12H),
   4.95 (d, J=5Hz, 1H),
   5.85 (dd, J=5Hz, J=9Hz, 1H),
   6.34 (d, J=9Hz, 1H),
   6.95 (s, 1H),
   7.23 - 7.44 (m, 15H)
X-ray powder diffraction pattern; 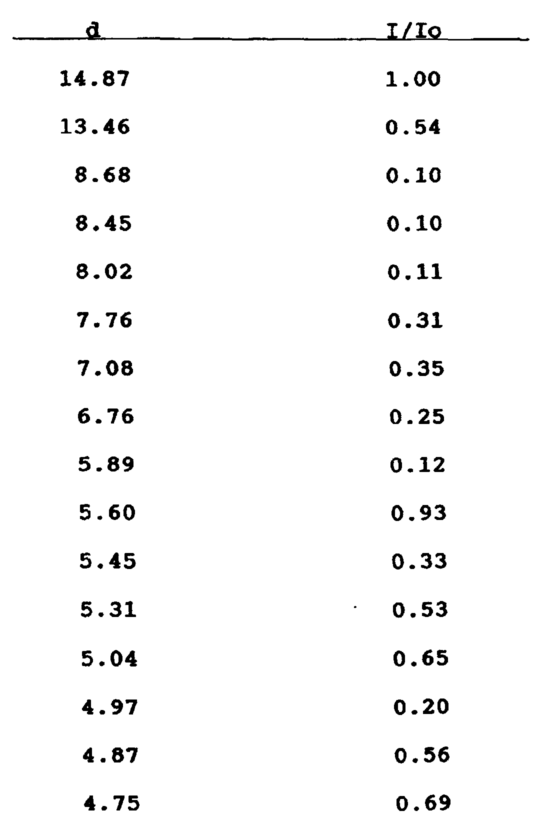 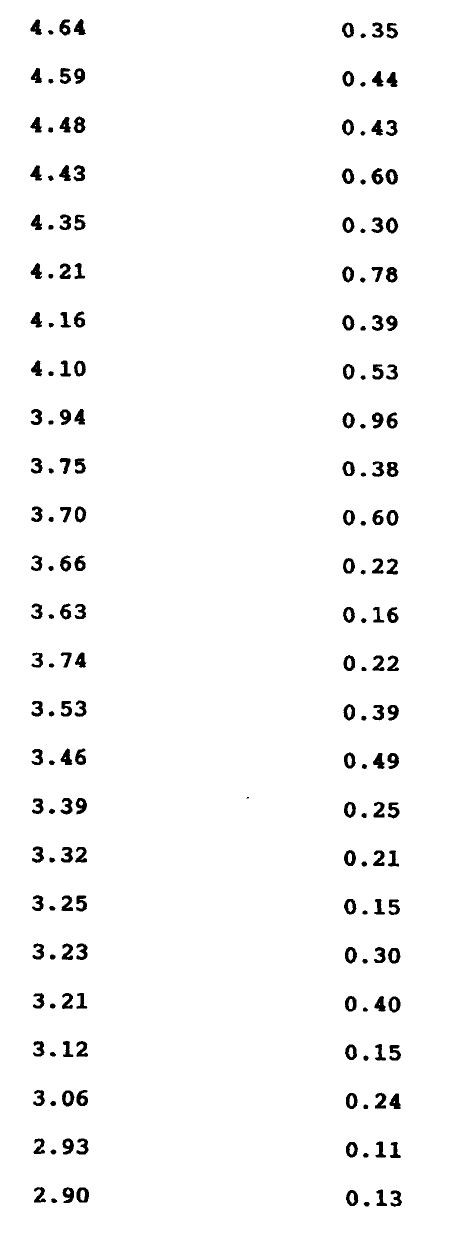 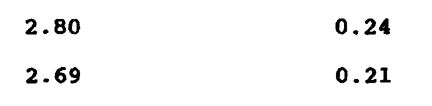 wherein d is the interplanar spacing, and I/Io is the relative intensity.

Subsequently, stability of Compound (1a) under crystallization conditions was tested. The results are shown in the following Comparative Test Examples 1 and 2.

### Comparative Test Example 1

200 mg of oily 7-phenylacetamido-3-chloromethyl-cephem-4-carboxylic acid p-methoxybenzyl ester was dissolved in 0.2 ml of dimethylformamide. The solution was pored into 1 ml of methanol precooled to 3°C to precipitate crystals. The mixture was stirred at 3 to 5°C for 20 hours. Then, an ethyl acetate/water mixture was added to dissolve the crystals, and the solution was subjected to extraction. The organic layer was dried over sodium sulfate. The resulting solution was filtered, and the filtrate was concentrated under reduced pressure. The concentrate was purified by silica gel chromatography (Wako gel C-200, benzene/ethyl acetate=4/1), giving 197 mg of oily 7-phenylacetamido-3-chloromethyl-cephem-4-carboxylic acid p-methoxybenzyl ester (yield: 99%).

### Comparative Test Example 2

200 mg of oily 7-phenylacetamido-3-chloromethyl-cephem-4-carboxylic acid p-methoxybenzyl ester was dissolved in 0.2 ml of dimethylformamide. The solution was pored into 1 ml of methanol to precipitate crystals. The mixture was stirred at 30 to 35°C for 20 hours. Then, an ethyl acetate/water mixture was added to dissolve the crystals, and the solution was subjected to extraction. The organic layer was dried over sodium sulfate. The resulting solution was filtered, and the filtrate was concentrated under reduced pressure. Thin-layer chromatography and NMR spectrum analysis of the obtained product revealed that various compounds other than the desired 7-phenylacetamido-3-chloromethyl-cephem-4-carboxylic acid p-methoxybenzyl ester were produced.

For example, thin-layer chromatography of the obtained product (conditions: Merk Pre-Coated TLC Plates SILICA GEL 60F254 (Merk); benzene/ethyl acetate=4/1) developed color in zones corresponding to 7-phenylacetamide-3-chloromethyl-cephem-4-carboxylic acid p-methoxybenzyl ester (Compound (1a)) (Rf=0.46), 7-phenylacetamide-3-methoxymethyl-cephem-4-carboxylic acid p-methoxybenzyl ester (Compound A) (Rf=0.33) and decomposition product of Compound (1a) (Rf≒0, in the vicinity of the placing point). Thus, the presence of Compound (1a), Compound A and decomposition product of Compound (1a) was demonstrated.

Then, the above filtrate concentrated under reduced pressure was purified by silica gel column chromatography (Wako gel C-200, benzene/ethyl acetate=4/1), giving 17 mg of a highly polar component, i.e., Compound A (yield: 9%) as well as 141 mg of crystals of Compound (1a) (yield: 71%).

The presence of decomposition product of Compound (1a) was also confirmed. The NMR spectrum analysis revealed that the peak corresponding to the lactam ring was eliminated, indicating that the decomposition product was a mixture of various compounds wherein the lactam ring of Compound (1a) had opened.

In the following Reference Examples, cefazolin was prepared from the cephalosporin crystals (7-phenylacetamido-3-chloromethyl-cephem-4-carboxylic acid p-methoxybenzyl ester crystals).

### Reference Example 1

### Preparation of 7-phenylacetamido-3-(2-methyl-1,3,4-thiadiazole-5-yl)thiomethyl-3-cephem-4-carboxylic acid p-methoxybenzyl ester (Compound (3))

10 g of 7-phenylacetamide-3-chloromethyl-cephem-4-carboxylic acid p-methoxybenzyl ester crystals (compound (1a)) and 100 ml of acetone were placed in a 200-ml four-necked flask and heated to 35°C with stirring. Most of the crystals were not dissolved but were formed into slurry. A previously prepared solution of 3.3 g of 5-methyl-2-mercapto-1,3,4-thiadiazole in 22.6 ml of an aqueous solution of 1N sodium hydroxide was added dropwise to the above mixture over a period of 20 to 30 minutes. When addition was completed, the reaction mixture was homogeneous. However, crystals were precipitated in several minutes. Ten minutes after completion of addition, 4.2 ml of 0.5N hydrochloric acid was added to the reaction mixture, followed by 10-minute stirring. The resulting reaction mixture was cooled 5°C or lower while adding 89 ml of water dropwise. The resulting mixture was aged for 1 hour with stirring. After aging, crystals of 7-phenylacetamido-3-(2-methyl-1,3,4-thiadiazole-5-yl)thiomethyl-3-cephem-4-carboxylic acid p-methoxybenzyl ester (compound (3)) was collected by suction filtration, washed with 10 ml of cold acetone and dried under reduced pressure, giving 11. 4 g of the title compound (yield: 95%).

### Reference Example 2

### Preparation of 7-amino-3-(2-methyl-1,3,4-thiadiazole-5-yl)thiomethyl-3-cephem-4-carboxylic acid (Compound (5))

60 ml of m-cresol and 0.24 ml of concentrated sulfuric acid were placed in a 200-ml four-necked flask, and heated to 35°C. Then, 10 g of the crystals of Compound (3) was added and reacted. The reaction was performed while maintaining the reaction temperature at 30 to 40°C and monitoring the reaction. The reaction time was about 2 to 3 hours. After completion of reaction, 200 ml of butyl acetate was added to the reaction mixture, which was then cooled to 5°C. After cooling, 65 ml of 4% aqueous sodium bicarbonate was added to the reaction mixture to extract 7-phenylacetamido-3-(2-methyl-1,3,4-thiadiazole-5-yl)thiomethyl-3-cephem-4-carboxylic acid (Compound (4)) into the aqueous layer. Further, 10 ml of water was added to the butyl acetate/m-cresol mixture to extract Compound (4) into the aqueous layer. The two aqueous solutions of extracted Compound (4) were mixed together, and 300 ml of butyl acetate was added to the solution mixture of extracted Compound (4) for washing, and then separated therefrom. The solution mixture was passed through an adsorption resin column (25 ml of Amberlite AXT-33 resin). The adsorption resin column was washed with 75 ml of water. The mixture of the treated solution of Compound (4) and the liquid obtained by washing the column was placed in an enzyme reactor containing 4 g of an enzyme (PGA-450, a product of Bellinger-Mannheim) to carry out a reaction at 28°C and at pH 7.7 to 8.1. The pH value was adjusted with 1N aqueous ammonia. The completion of the reaction was ascertained by the end of ammonia consumption. The enzyme was filtered off, and the reaction product was washed. The obtained solution of 7-emino-3-(2-methyl-1,3,4-thiadiazole-5-yl)thiomethyl-3-cephem-4-carboxylic acid (Compound (5)) was cooled to 5°C or lower and adjusted to pH 3.8 with 3N hydrochloric acid. After adjusting the pH value, the solution was aged at 5°C or lower for 1 hour. Then, the crystals of Compound (5) were collected by filtration, washed successively with 20 ml of cold water and 20 ml of acetone, and dried under reduced pressure, giving 5.2 g of the title compound (yield from Compound (3): 88%).

### Reference Example 3

### Synthesis of cefazolin

### (1) Preparation of acid anhydride mixture

3.72 g of 1H-tetrazole-1-acetic acid and 40 ml of methylene chloride were placed in a 100-ml four-necked flask. 2.94 g of triethylamine was added to the methylene chloride solution, and the resulting mixture was cooled to -10C. At -10°C or lower, 3.32 g of pivalic acid chloride was added, and the reaction mixture was adjusted to 0°C and aged at the same temperature for 1 hour.

### (2) Preparation of solution of Compound (5) in methylene chloride

4.3 g of diisopropylamine and 30 ml of methylene chloride were placed in a 100-ml four-necked flask. 2.94 g of Compound (5) was added to and dissolved in the methylene chloride solution, and the resulting solution was cooled to -20°C or lower.

### (3) Reaction for preparing cefazolin

The methylene chloride solution of Compound (5) was added dropwise to the acid anhydride mixture prepared above, over a period of 20 to 30 minutes at -20°C or lower. After addition, coaling was stopped and the mixture was stirred at room temperature for 30 minutes. The completion of the reaction was ascertained, and 60 ml of water was added for extracting cefazolin. Further, 40 ml of water was added to the methylene chloride layer to extract cefazolin again. The two solutions of cefazolin extract were mixed together, and the resulting aqueous solution was adjusted to pH 4.5. 30 ml of methylene chloride was added to the solution to wash the solution of cefazolin extract, and then separated therefrom. 1.5 g of activated carbon was added to the cefazolin solution, followed by 15-minute stirring. Thereafter, the activated carbon was filtered off. The filtrate was adjusted to pH 2 with 3N hydrochloric acid solution to precipitate crystals, and aged at 5°C or lower for 1 hour. After aging, cefazolin crystals were collected by filtration, washed with 20 ml of cold water and dried under reduced pressure, giving 5.92 g of cefazolin crystals (yield: 90%).

## Claims

1. Cephalosporin crystals represented by the formula: 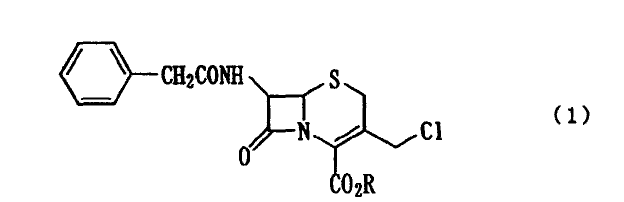 wherein R represents p-methoxybenzyl or diphenylmethyl.

2. Cephalosporin crystals according to Claim 1 wherein R represents p-methoxybenzyl.

3. Cephalosporin crystals according to Claim 2 having the following X-ray powder diffraction pattern obtained by copper radiation of λ=1.5418 angstroms through a monochromator silk filter:
| d | I/Io |
|---|---|
| 12.94 - 12.96 | 0.55 - 0.67 |
| 11.67 - 11.69 | 0.46 - 0.56 |
| 9.90 - 9.92 | 0.09 - 0.11 |
| 6.46 - 6.48 | 0.57 - 0.69 |
| 6.32 - 6.34 | 0.22 - 0.26 |
| 5.80 - 5.82 | 0.56 - 0.68 |
| 4.95 - 4.97 | 0.42 - 0.52 |
| 4.72 - 4.74 | 0.63 - 0.77 |
| 4.65 - 4.67 | 0.65 - 0.79 |
| 4.50 - 4.52 | 0.60 - 0.74 |
| 4.43 - 4.45 | 0.28 - 0.34 |
| 4.28 - 4.30 | 0.50 - 0.62 |
| 4.16 - 4.18 | 1.00 |
| 4.04 - 4.06 | 0.23 - 0.28 |
| 3.97 - 3.99 | 0.15 - 0.19 |
| 3.85 - 3.87 | 0.67 - 0.81 |
| 3.77 - 3.79 | 0.42 - 0.52 |
| 3.69 - 3.71 | 0.13 - 0.15 |
| 3.55 - 3.57 | 0.06 - 0.08 |
| 3.53 - 3.54 | 0.05 - 0.07 |
| 3.44 - 3.46 | 0.49 - 0.59 |
| 3.36 - 3.38 | 0.18 - 0.22 |
| 3.28 - 3.30 | 0.13 - 0.15 |
| 3.21 - 3.23 | 0.18 - 0.22 |
| 3.19 - 3.21 | 0 14 - 0.17 |
| 3.15 - 3.17 | 0.18 - 0.22 |
| 3.08 - 3.10 | 0.16 - 0.20 |
| 2.89 - 2.91 | 0.20 - 0.24 |
| 2.84 - 2.86 | 0.09 - 0.11 |
| 2.67 - 2.69 | 0.13 - 0.15 |
| 2.59 - 2.61 | 0.14 - 0.18 |
| 2.57 - 2.59 | 0.10 - 0.12 |
| 2.49 - 2.51 | 0.09 - 0.11 |
| 2.48 - 2.50 | 0.12 - 0.14 |
wherein d is the interplanar spacing, and I/Io is the relative intensity.

4. Cephalosporin crystals according to Claim 1 wherein R is diphenylmethyl.

5. Cephalosporin crystals according to Claim 4 having the following X-ray powder diffraction pattern obtained by copper radiation of λ=1.5418 angstroms through a monochromator silk filter: 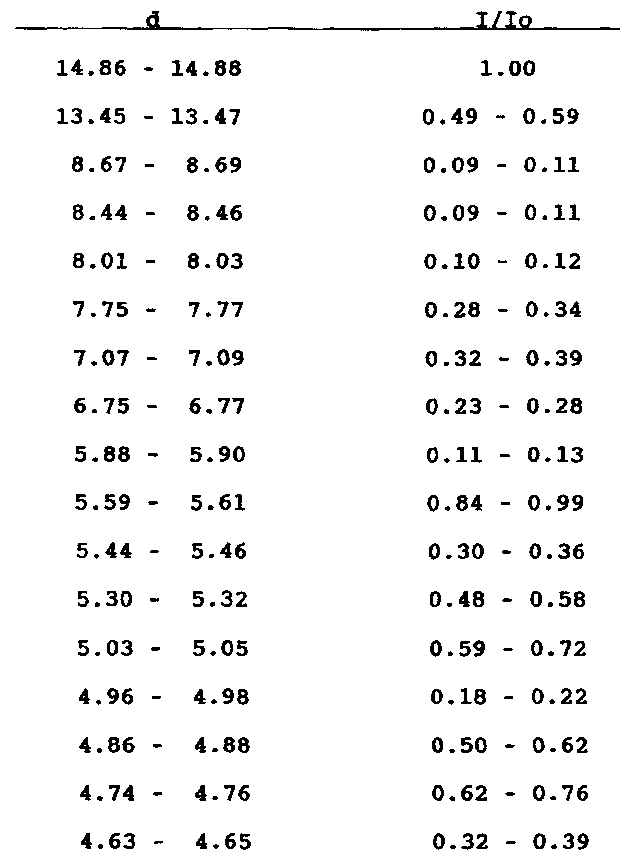 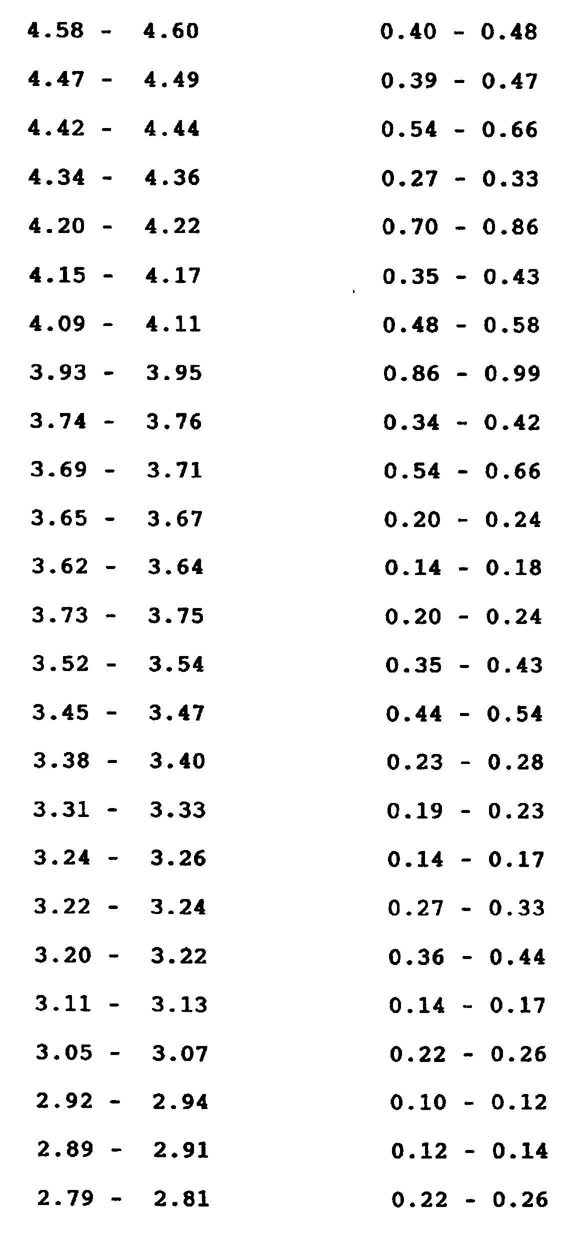 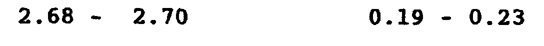 wherein d is the interplanar spacing, and I/Io is the relative intensity.

6. A process for preparing cephalosporin crystals represented by the formula: 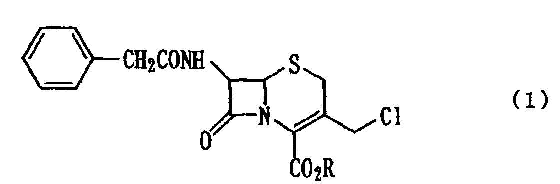 wherein R is p-methoxybenzyl or diphenylmethyl, the process comprising the step of crystallizing, in a solvent containing an alcohol, an oily cephalosporin represented by the formula: 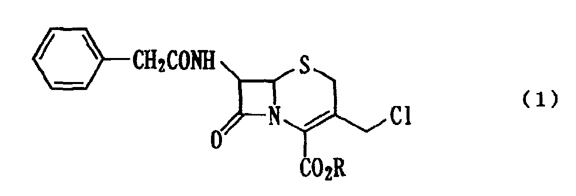 wherein R is as defined above.

7. A process for preparing cephalosporin crystals according to Claim 6 wherein the alcohol is an aliphatic saturated lower alcohol.

8. A process for preparing cephalosporin crystals according to Claim 7 wherein the aliphatic saturated lower alcohol is at least one member selected from the group consisting of methanol, ethanol and isopropanol.

9. A process for preparing cephalosporin crystals according to Claim 6 wherein a solution of an oily cephalosporin in a solvent is added to a cooled alcohol to precipitate cephalosporin crystals.

10. A process for preparing cephalosporin crystals according to Claim 9 wherein the temperature of the cooled alcohol is -20 to 15°C.

11. A process for preparing cephalosporin crystals according to Claim 9 wherein the temperature of the cooled alcohol is 0 to 5°C.

12. A process for preparing cephalosporin crystals according to Claim 9 wherein the weight ratio of the solvent for dissolving the oily cephalosporin to the alcohol is 100:100 to 100:10000.

13. A process for preparing cephalosporin crystals according to Claim 9 wherein the weight ratio of the solvent for dissolving the oily cephalosporin to the alcohol is 100:200 to 100:3000.

14. A process for preparing cephalosporin crystals according to Claim 9 wherein the weight ratio of the solvent for dissolving the oily cephalosporin is 100:400 to 100:1000.
